# EUROPÄISCHE PATENTSCHRIFT

(11) **EP 3 524 317 B1**
(45) Veröffentlichungstag und Bekanntmachung des Hinweises auf die Patenterteilung: **24.03.2021**
(21) Anmeldenummer: 19164931.8
(22) Anmeldetag: 16.01.2013
(51) Int. Cl.: A61N 1/32, A61N 1/04, A61H 7/00, A61H 23/02

(54) **IN EINER HAND HALTBARES GERÄT ZUR ELEKTRISCH UNTERSTÜTZTEN HAUTBEHANDLUNG**
APPARATUS FOR ELECTRICALLY ASSISTED SKIN TREATMENT WHICH CAN BE HELD
APPAREIL POUVANT ÊTRE TENU DANS LA MAIN DESTINÉ AU TRAITEMENT DE LA PEAU ASSISTÉ ÉLECTRIQUEMENT

(30) Priorität: 16.01.2012 DE 102012000563; 14.05.2012 DE 102012009514
(43) Veröffentlichungstag der Anmeldung: 14.08.2019
(62) Teilanmeldung aus: 13710970.8
(73) Patentinhaber: Swiss Spa System Ltd., Hong Kong (CN)
(72) Erfinder: Doyle, James N., Westlake, OH 44145 (US); Gimelli, Bruno, 3052 Zollikofen (CH)
(74) Vertreter: Hebing, Norbert

(56) Entgegenhaltungen:
- EP-A1- 2 384 707
- EP-A2- 2 277 585
- WO-A1-2005/087308
- US-B1- 6 385 487

## Beschreibung

Die Erfindung bezieht sich auf ein in einer Hand haltbares Gerät zur elektrisch unterstützten Hautbehandlung, aufweisend:
Ein Gehäuse,
eine erste äußere Elektrode am Gehäuse, die in Kontakt zur Hand steht, wenn das Gerät vom Nutzer zum Gebrauch in der Hand gehalten wird,
eine zweite äußere Elektrode am Gehäuse, die auf den zu behandelnden Hautbereich aufsetzbar ist, und
eine elektrische Spannungsquelle in Form von Batterien innerhalb des Gehäuses, deren Pole beim Betrieb des Gerätes mit den Elektroden in einer elektrischen Verbindung stehen.

Ein derartiges Gerät ist in der WO 2005/087308 A1 beschrieben. Um das Gerät einzusetzen, nimmt der Nutzer es in die Hand und setzt eine als Elektrode fungierende Kappe auf die zu behandelnden Hautbereiche auf. Mit der ersten Elektrode wird ein elektrisch leitender Kontakt zur Hand und mit der zweiten Elektrode ein elektrisch leitender Kontakt zu dem zu behandelnden Hautbereich hergestellt. Da die beiden Elektroden an je einem Pol einer elektrischen Energiequelle angeschlossen sind, entsteht ein elektrischer Stromkreis, der den Körper des Nutzers mit einschließt, wobei je nach Polung ein positiver oder ein negativer elektrischer Strom von der Kappe in den zu behandelnden Hautbereich einfließt.

Dies kann dazu benutzt werden, die Wirkungsweise von Behandlungscremes bzw. Reinigungscremes zu erhöhen, da die Wirkstoffe einer Behandlungscreme mit dem elektrischen Strom in die Haut transportiert werden und bei einer entgegengesetzten Polung Schmutzstoffe aus der Haut in eine Reinigungscreme transportiert werden.

Das Gerät ist weiterhin mit einer Vielzahl von auswechselbaren Kappen versehen, deren Form jeweils an die zu behandelnde Hautoberfläche angepasst ist. Es gibt eine erste Kappe mit einer relativ glatten Oberfläche, eine zweite Kappe, die kammartig gestaltet ist, die vor allem zur Behandlung der Kopfhaut eingesetzt werden kann, und eine dritte Kappe mit einer welligen Struktur, womit ein Massageeffekt verbunden ist. Mit dem Gerät werden gute Ergebnisse sowohl bei der Hautreinigung als auch bei der Einbringung von Wirkstoffen erzielt. Dies soll aber noch weiter effektiviert werden.

Die EP 2 384 707 A1 beschreibt ein Zungenreinigungsgerät, das einen zylindrischen Griffkörper zum Greifen des Geräts aufweist. In der axialen Verlängerung des Griffkörpers befindet sich ein in Schwingung versetzbarer Kopf, dessen flache Außenseite mit Kontaktelektroden und mechanischen Abriebhilfen versehen ist.

Die US 6,385,487 B1 beschreibt ein Gerät zum Einbringen von Wirkstoffen in die Haut. Auch dieses besteht aus einem zylindrischen Griffkörper, dessen eine Stirnseite als Topfelektrode ausgebildet ist, in dem sich ein Schwamm befindet, der mit den einzubringenden Wirkstoffen getränkt ist.

Die EP 2 277 585 A2 beschreibt einen Wirkstoff enthaltenden und eine Gitterstruktur aufweisenden Streifen, der über die Stirnseite eines zylinderförmigen Griffkörpers gespannt wird.

Die Erfindung beruht somit auf der Aufgabe, die Wirkungsweise des Gerätes noch weiter zu verbessern.

Zur Lösung der Aufgabe wird vorgeschlagen, dass
das Gehäuse ein flaches Gehäuse mit einer Unter- und einer Oberseite ist,
die zweite Elektrode sich an der Unterseite des Gehäuses befindet, wobei sie fast die gesamte Unterseite einnimmt,
die erste Elektrode sich umlaufend am Außenrand der Oberseite des Gehäuses befindet,
sich innerhalb des Gehäuses ein Vibrator befindet und
der Vibrator zentral oberhalb der zweiten Elektrode mittels eines Sockels an der unteren Wand des Gehäuses angebracht ist und somit die Wand zusammen mit der zweiten Elektrode in Schwingung versetzt, wenn er in Betrieb genommen wird, so dass beim Betrieb des Gerätes ein verstärkter Massageeffekt auf der zu behandelnden Haut entsteht, wodurch die Hautporen geweitet werden.

Ein solcher Vibrator setzt das Gerät in eine schwingende Bewegung, so dass beim Betrieb des Gerätes ein verstärkter Massageeffekt auf der zu behandelnden Haut entsteht, wodurch die Hautporen geweitet werden und ein noch besserer Übergang der Schmutzstoffe aus der Haut bzw. der Wirkstoffe in die Haut realisiert wird.

Der Massageeffekt ist besonders ausgeprägt, wenn der Vibrator in der Nähe der zweiten äußeren Elektrode angebracht ist, weil dann der Bereich des Gerätes besonders stark vibriert, der sich an dem zu behandelnden Hautbereich befindet.

Vorzugsweise wird die Erfindung bei einem Gerät eingesetzt, bei dem sich auf dessen Oberseite ein Display und eine Taste zur Auswahl eines Behandlurigsprogramms befinden.

Bei der Energiequelle kann es sich um Batterien oder auch um Akkus handeln, die in ein Fach des Basiskörpers eingelegt werden. Soweit für das Gerät Akkus vorgesehen sein sollen, ist es denkbar, zu dem Gerät auch ein Ladegerät zu liefern, mit dem die Akkus im Gerät aufgeladen werden können.

Da - wie oben erläutert - das Gerät vorsieht, dass die Polarität der Elektroden austauschbar ist, hat dies zur Folge, dass auch die Polarität der Stromversorgung des Vibrators sich umkehrt. Daher muss der Vibrator einen elektrischen Antrieb besitzen, der unempfindlich ist gegenüber einer Vertauschung der Polarität. Im einfachsten Fall handelt es sich bei dem Vibrator um einen elektrischen Motor, an dessen Welle ein Excenter befestigt ist. Denkbar wären aber auch Schwingungserreger, bei denen eine Masse durch Elektromagnete in schneller Folge mal in die eine, mal in die andere Richtung gezogen wird.

Um den Aufbau des Gerätes einfach zu halten, ist vorgesehen, dass eine vom Nutzer des Gerätes einstellbare Schalteinrichtung im Gehäuse vorhanden ist, die die elektrischen Verbindungen der Energiequelle zu den Elektroden herstellt, und dass auch der Vibrator über die Schalteinrichtung mit der elektrischen Energiequelle verbunden ist. Es liegt somit eine gemeinsame Energiequelle für die Elektrode und den Vibrator vor.

Vorzugsweise ist die Schalteinrichtung mit einem elektronischen Speicher versehen, in dem die Parameter von auszuwählenden Betriebszuständen gespeichert sind, die vom Benutzer mittels einer Taste auswählbar sind, wobei einer der Parameter sich auf die Polarität der Elektroden bezieht, und wobei der Vibrator von der Art ist, dass er unabhängig von der Polarität der angelegten Spannung arbeitet.

Damit die Batterien möglichst selten geladen werden müssen, muss dafür gesorgt werden, dass der Stromverbrauch des Gerätes möglichst minimiert wird. Dies ist insbesondere dann der Fall, wenn das Gerät mit einem Vibrator versehen ist, der die zweite Elektrode, die auf die zu behandelnde Hautfläche aufgesetzt wird, in eine rüttelnde Bewegung versetzt. Um bei einem solchen Gerät den Stromverbrauch zu minimieren, sieht die Erfindung vor, dass eine Einrichtung zum Detektieren eines Stromflusses durch die zweite Elektrode, ein Schalter in der Verbindung des Antriebs des Vibrators zu Batterien und eine Steuerung vorgesehen sind, wobei die Steuerung so eingerichtet ist, dass der Schalter nur dann geschlossen ist, wenn durch die zweite Elektrode ein Strom fließt.

Mit anderen Worten, der Vibrator wird nur dann eingeschaltet, wenn die zweite Elektrode auf die zu behandelnde Hautfläche aufgesetzt ist und durch sie ein Strom durch die Haut fließt. Dieser Stromfluss wird detektiert und von einer Steuerung genutzt, um den Schalter zu betätigen. Die Einrichtung lässt sich elektronisch realisieren. Dies bedeutet, dass der Schalter als ein Transistor ausgeführt ist.

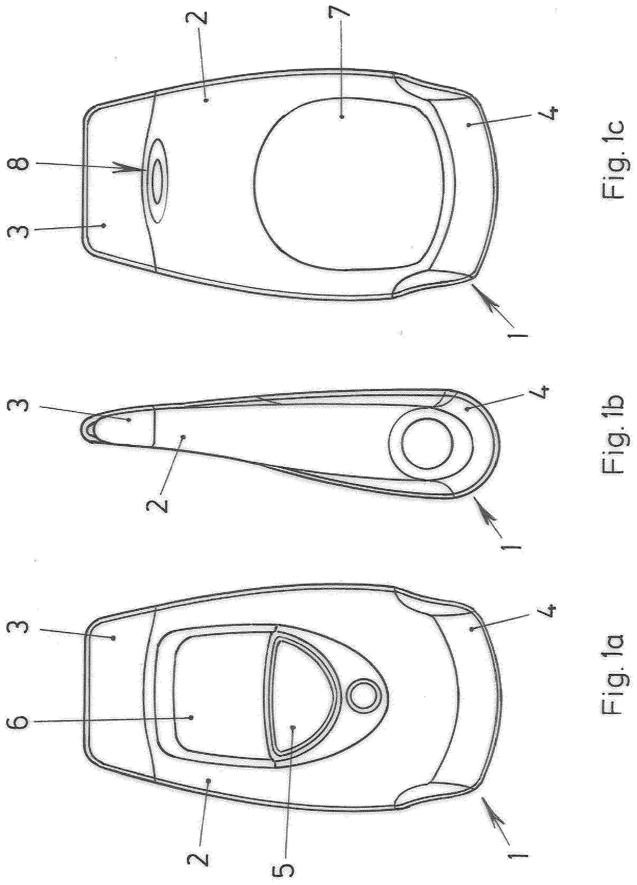

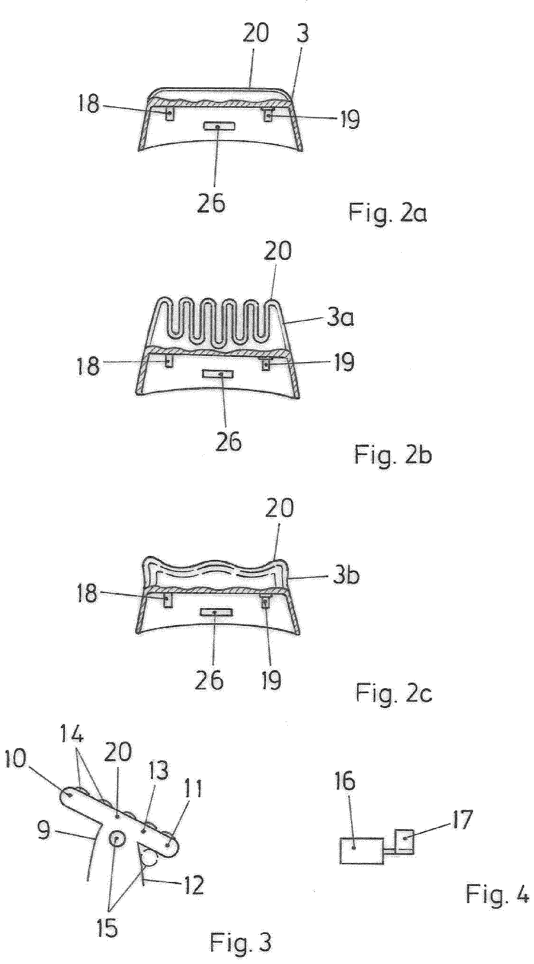

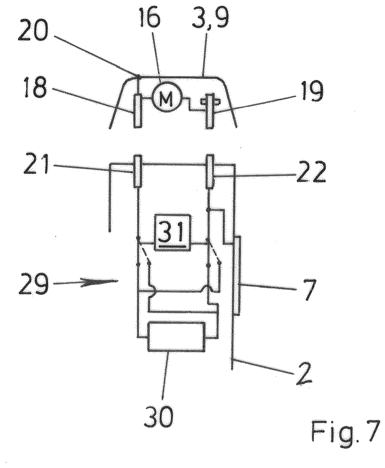

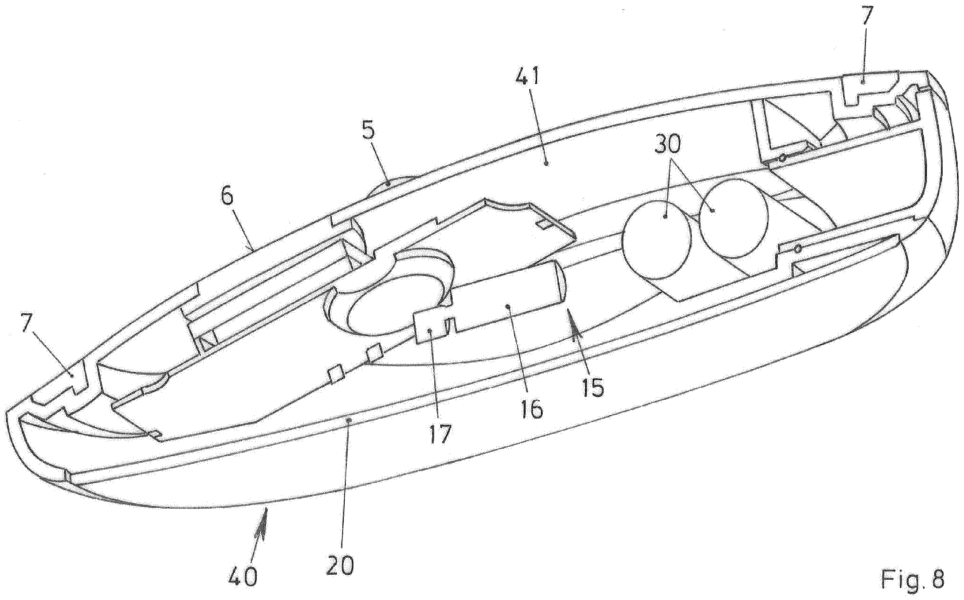

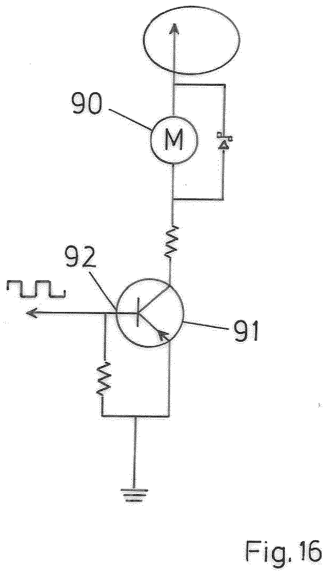

Im Folgenden soll anhand eines Ausführungsbeispiels, dargestellt in der Fig. 8, die Erfindung näher erläutert werden. Dabei beziehen sich die zusätzlichen Figuren 7 und 16 auf die mögliche Verschaltung der Elektroden und die Fig. 4 auf den Vibrator. Die Ausführungen nach den Fig. 1-3 sind für sich genommen nicht Gegenstand der Ansprüche. Es zeigen:
- Fig.1a, b, c: die Ansichten eines Gerätes, wobei als Kappe eine gerundete Struktur vorgesehen ist,
- Fig.2a, b, c: verschiedene Kappenformen zur Verwendung mit dem Gerät,
- Fig. 3: den Querschnitt einer Kappenform, die für den Betrieb mit einem Vibrator besonders geeignet ist,
- Fig. 4: einen Motor mit einem Excenter,
- Fig. 7: einen Schaltkreis zum Betrieb des Gerätes,
- Fig. 8: eine Ausführung des erfindungsgemäßen Gerätes,
- Fig. 16: eine Schaltung für die Steuerung eines Vibrationsmotors.

Zunächst wird auf die Fig. 1 Bezug genommen. Wie die drei Ansichten zeigen, ist das Gerät 1 etwa handtellergroß und hat eine rechteckige, flache Form. Es besteht aus einem Basiskörper 2 und einer darauf aufgesteckten Kappe 3. An der unteren kurzen Seite des Basiskörpers 2 befindet sich ein Fach 4 zur Aufnahme von Batterien, die als Energiequelle zum Betrieb des Gerätes 1 fungieren.

An der gegenüberliegenden oberen, kurzen Seite des Basiskörpers 2, die nicht so breit wie die untere Seite ist, ist die auswechselbare Kappe 3 aufgesteckt. Auf der Vorderseite des Basiskörpers 2 ist eine Taste 5 zur Auswahl eines Behandlungsprogramms angeordnet, wobei das ausgewählte Behandlungsprogramm in einem darüber liegenden Display 6 angezeigt wird. Auf der Rückseite befindet sich eine großflächige erste Elektrode 7 und darüber ein Druckknopf 8 zur Auslösung einer Raste, mit der die Kappe 3 am Basiskörper 2 gehalten wird.

In der Fig. 2 sind im Längsschnitt verschiedene Kappenformen dargestellt. Die Fig. 2a zeigt die schon in der Fig. 1 dargestellte Kappe 3, diese hat eine gerundete obere Abschlusskante.

Die Kappe 3a gemäß der Fig. 2b hat eine kammartige Struktur und eignet sich daher besonders zur Behandlung der Kopfhaut. Die Fig. 2c zeigt eine Kappe 3b mit einer wellenförmig ausgeführten Kante, wodurch der Druck auf die Haut punktuell verstärkt werden kann, so dass beim Hin- und Hergehen der Kappe 3b auf der Haut ein Massageeffekt entsteht.

Gemäß der Fig. 3 ist im Querschnitt eine weitere Kappe 9 dargestellt, die eine Abwandlung der Kappe 3 gemäß Fig. 2a darstellt. An der oberen Kante der Kappe 9 schließen sich zwei Zungen 10, 11 an, die zusammen eine gegenüber einer Basis 12 der Kappe 9 schräg gestellte Plattform 13 bilden. Die Oberseite der Plattform 13 bildet eine durchgehende Fläche, die auf die Haut aufsetzbar ist und die mit Rippen 14 versehen ist.

Diese Form ist besonders geeignet zur Verbindung mit einem Vibrator 15. Dieser ist entweder vorzugsweise im spitzen Winkel zwischen der einen Zunge 11 und der Basis 12 der Kappe 9 angebracht (strichpunktiert) oder im Kopf der Basis 12 der Kappe 9 (durchgehende Linie). Denkbar wäre auch eine Unterbringung in einer der Zungen 10, 11.

Der Vibrator 15 besteht im einfachsten Fall gemäß der Fig. 4 aus einem Elektromotor 16, an dessen Welle ein Excenter 17 angebracht ist. Die Ausrichtung der Welle im Bezug zum Gerät ist beliebig. Möglich ist eine Ausrichtung parallel zur oberen Kante der Kappe 9.

Wie man den Fig. 2a bis 2c entnehmen kann, befinden sich innerhalb der Kappe 3, 3a, 3b zwei metallische Kontaktstifte 18, 19, wobei der eine Kontaktstift 18 mit der Kappe 3 in elektrischer Verbindung steht und der andere Kontaktstift 19 isoliert gegenüber der Kappe 3, 3a, 3b ist. Die Kappen 3, 3a, 3b bestehen aus einem leitfähigen Material oder besitzen zumindest einen Überzug aus einem leitfähigen Material, das die zweite Elektrode 20 bildet. Der erste Kontaktstift 18 steht somit in Verbindung zu diesem Material, während der zweite Kontaktstift 19 dazu isoliert ist.

In der Fig. 7 ist schematisch ein Schaltplan dargestellt. Der Antrieb des Vibrators, also im Ausführungsbeispiel der Elektromotor 16, ist zwischen den beiden Kontaktstiften 18, 19 in der Kappe 3, 9 geschaltet, während der erste Kontaktstift 18 nur mit der zweiten Elektrode 20 an der Kappe 3, 9 verbunden ist.

Am Basiskörper 2 befinden sich zwei Gegenstifte 21, 22, die über eine Schalteinrichtung in Form eines Wechselschalters 29 mit einer Spannungsquelle 30 (Batterien) verbunden sind. Der Gegenstift 21, der mit dem isolierten Kontaktstift 18 in der Kappe 3, 9 in Kontakt tritt, hat eine Verbindung zur ersten Elektrode 7 am Basiskörper 2.

Wird nun die Kappe 3, 9 - wie in der Fig. 7 angedeutet - auf den Basiskörper 2 aufgesteckt (eine Codierung verhindert dabei eine Vertauschung der Kontakte), entsteht ein erster Stromkreis, bestehend aus der Spannungsquelle 30 und dem Elektromotor 16, der über die beiden Stiftpaare verläuft, sowie im Betrieb ein zweiter Stromkreis, der über den ersten Kontakt der zweiten Elektrode 20 über den Körper des Nutzers zur ersten Elektrode 7 und zurück zur Spannungsquelle 30 verläuft.

Der Wechselschalter ist in Form einer Transistorschaltung realisiert, die von einer Steuereinrichtung 31 angesteuert wird. Die Steuereinrichtung 31 enthält einen elektronischen Speicher, in dem mehrere Betriebsarten abgespeichert sind. Die Betriebsarten werden durch die Parameter (Dauer der Behandlung, Polarisierung der Elektroden, Zuschaltung des Vibrators) bestimmt. Der Nutzer kann durch Betätigung der Taste 5 eine bestimmte Betriebsart auswählen, die in dem Display 6 angezeigt wird.

Eine Hautbehandlung besteht in der Regel aus einem ersten Schritt, bei dem zunächst eine Reinigungscreme aufgebracht wird und das Gerät in einer ersten Polung betrieben wird, wobei durch den Stromfluss Schadstoffe aus der Haut in die Reinigungscreme übergehen. Hierbei werden Kappen 3 genutzt, die keinen Vibrator enthalten, weil die Creme gerade nicht in die Haut eingearbeitet werden soll.

In einem zweiten Schritt, nachdem die Reinigungscreme entfernt wurde, wird eine Behandlungscreme aufgetragen und das Gerät 1 in einer zweiten Polung betrieben, so dass die Wirkstoffe aus der Behandlungscreme in die Haut eindringen können. Bei dieser Vorgehensweise werden Kappen 9 benutzt, die einen Vibrator 15 aufweisen, so dass neben dem Stromeffekt, der die Wirkstoffe in die Haut transportiert, auch ein Einmassageeffekt vorhanden ist, mit dem der Wirkstoff in die Haut einmassiert wird. Neben der erhöhten Effektivität wird auch ein angenehmes Behandlungsgefühl erzeugt, da eine vibrierende Massage vom Nutzer als angenehm empfunden wird.

Die Fig. 8 zeigt im Querschnitt ein Gerät 40, das insbesondere zur Zellulitis-Behandlung von großen Hautflächen geeignet ist.

An der Unterseite eines flachen Gehäuses 41 befindet sich eine fast die gesamte Unterseite einnehmende zweite Elektrode 20, die auf die zu behandelnde Hautfläche aufgesetzt wird. Auf der Oberseite befinden sich ein Display 6 und eine Taste 5 zur Auswahl eines Behandlungsprogramms.

Am Außenrand der Oberseite des Gehäuses 41 befindet sich umlaufend eine erste Elektrode 7, die in Kontakt zu der das Gerät 40 haltenden Hand steht. Innerhalb des Gehäuses 41 befinden sich eine Spannungsquelle 30 in Form von Batterien, sowie ein Vibrator 15, der aus einem Elektromotor 16 mit einem Excenter 17 besteht. Der Vibrator 15 ist zentral oberhalb der zweiten Elektrode 20 mittels eines nicht näher dargestellten Sockels an der unteren Wand des Gehäuses 41 angebracht und setzt somit die Wand zusammen mit der zweiten Elektrode 20 in Schwingung, wenn er in Betrieb genommen wird.

Die Verschaltung der Elektroden 7, 20 des Elektromotors 16 und der Spannungsquelle 30 entspricht der, die in Fig. 7 gezeigt ist, wobei allerdings die Stifte 18, 19; 21, 22 durch durchgehende Verbindungen ersetzt sind. Die Stifte werden nicht benötigt, da das Gerät 40 in dieser Ausführung keine auswechselbaren Kappen 3, 9 mit zweiten Elektroden 20 besitzt.

Wie man der Fig. 16 entnehmen kann, ist der Antrieb 90 eines Vibrators in Reihe geschaltet mit der Kollektor/Emitter/Strecke eines Transistors 91. An der Basis 92 des Transistors 91 wird eine Steuerspannung angelegt. Liegt diese vor, schaltet der Transistor durch, so dass der Motor Strom durchflossen ist. Liegt keine Spannung an der Basis 92 an, sperrt der Transistor 91, so dass der Antrieb 90 abgeschaltet wird.

Die Schaltspannung an der Basis wird von einer Steuerschaltung geliefert, die mittels eines Detektors feststellt, ob ein Strom zur zweiten Elektrode fließt.

### Bezugszeichenliste

- 1: Gerät
- 2: Basiskörper
- 3: Kappe
- 3a: Kappe
- 3b: Kappe
- 4: Fach
- 5: Taste

- 6: Display
- 7: erste Elektrode
- 8: Druckknopf
- 9: Kappe
- 10: Zunge

- 11: Zunge
- 12: Basis einer Kappe
- 13: Plattform
- 14: Rippen
- 15: Vibrator

- 16: Elektromotor
- 17: Excenter
- 18: Kontaktstift
- 19: Kontaktstift
- 20: zweite Elektrode

- 21: Gegenstift
- 22: Gegenstift
- 26: Schlitz
- 29: Wechselschalter
- 30: Spannungsquelle

- 40: Gerät
- 41: Gehäuse
- 90: Antrieb
- 91: Transistor
- 92: Basis

## Patentansprüche

1. In einer Hand haltbares Gerät zur elektrisch unterstützten Hautbehandlung, aufweisend:
Ein Gehäuse (41),
eine erste äußere Elektrode (7) am Gehäuse (41), die in Kontakt zur Hand steht, wenn das Gerät vom Nutzer zum Gebrauch in der Hand gehalten wird,
eine zweite äußere Elektrode (20) am Gehäuse (41), die auf den zu behandelnden Hautbereich aufsetzbar ist, und
eine elektrische Spannungsquelle in Form von Batterien (30) innerhalb des Gehäuses (41), deren Pole beim Betrieb des Gerätes mit den Elektroden (7, 20) in einer elektrischen Verbindung stehen,
**dadurch gekennzeichnet, dass**
das Gehäuse ein flaches Gehäuse (41) mit einer Unter- und einer Oberseite ist,
die zweite Elektrode (20) sich an der Unterseite des Gehäuses (41) befindet, wobei sie fast die gesamte Unterseite einnimmt,
die erste Elektrode (7) sich umlaufend am Außenrand der Oberseite des Gehäuses (41) befindet,
sich innerhalb des Gehäuses ein Vibrator (15) befindet und
der Vibrator (15) zentral oberhalb der zweiten Elektrode (20) mittels eines Sockels an der unteren Wand des Gehäuses (41) angebracht ist und somit die Wand zusammen mit der zweiten Elektrode (20) in Schwingung versetzt, wenn er in Betrieb genommen wird, so dass beim Betrieb des Gerätes ein verstärkter Massageeffekt auf der zu behandelnden Haut entsteht, wodurch die Hautporen geweitet werden.

2. Gerät nach Anspruch 1, **dadurch gekennzeichnet, dass** der Vibrator (15) in der Nähe der zweiten äußeren Elektrode (20) angebracht ist.

3. Gerät nach Anspruch 1 oder 2 **dadurch gekennzeichnet, dass** sich auf der Oberseite ein Display (6) und eine Taste (5) zur Auswahl eines Behandlungsprogramms befinden.

4. Gerät nach einem der vorhergehenden Ansprüche, **dadurch gekennzeichnet, dass** der Vibrator (15) aus einem elektrischen Motor (16) besteht, an dessen Welle ein Excenter (17) befestigt ist.

5. Gerät nach einem der vorhergehenden Ansprüche, **dadurch gekennzeichnet, dass** eine vom Nutzer des Gerätes einstellbare Schalteinrichtung im Gehäuse (41) vorhanden ist, die die elektrischen Verbindungen der Energiequelle zu den Elektroden (7, 20) herstellt, dass auch der Vibrator (15) über die Schalteinrichtung mit der elektrischen Energiequelle verbunden ist, dass die Schalteinrichtung mit einem elektronischen Speicher versehen ist, in dem die Parameter von auszuwählenden Betriebszuständen gespeichert sind, die vom Benutzer mittels einer Taste (5) auswählbar sind, wobei einer der Parameter sich auf die Polarität der Elektroden bezieht, und dass der Vibrator (15) von der Art ist, dass er unabhängig von der Polarität der angelegten Spannung arbeitet.

6. Gerät nach einem der vorhergehenden Ansprüche, **dadurch gekennzeichnet, dass** eine Einrichtung zum Detektieren eines Stromflusses durch die zweite Elektrode (20), ein Schalter in der Verbindung des Antriebs des Vibrators zu Batterien und eine Steuerung vorgesehen sind, wobei die Steuerung so eingerichtet ist, dass der Schalter nur dann geschlossen ist, wenn durch die zweite Elektrode (20) ein Strom fließt.

## Claims

1. A hand-held device for electrically powered skin treatment, comprising:
a housing (41),
a first outer electrode (7) disposed on the housing (41), the first outer electrode being in contact with the hand when the device is held in the user's hand for use,
a second outer electrode (20) disposed on the housing (41), the second outer electrode (20) being placeable on the area of skin to be treated, and
an electrical energy source in the form of batteries (30) contained in the housing, the poles of the batteries being electrically connected to electrodes (7, 20) during operation of the device,
**characterized in that**
the housing is a shallow housing (41) having an underside and an upper side,
the second electrode (20) is located on the underside of the housing (41) and takes up almost the entire underside,
the first electrode (7) is located peripherally on the outer edge of the upper side of the housing (41),
a vibrator (15) is located within the housing (41), and that
the vibrator (15) is fixed by a socket on the lower wall of the housing (41) centrally above the second electrode (20) and thus vibrates the wall together with the second electrode (20) when operated so that during operation of the device, the device produces an intensified massaging effect on the skin to be treated, whereby the pores of the skin are widened.

2. The device according to claim 1, **characterized in that** the vibrator (15) is fixed in the vicinity of the second outer electrode (20).

3. The device according to claim 1 or 2, **characterized in that** a display (6) and a button (5) for selecting a treatment program are located on the upper side.

4. The device according to any one of the preceding claims, **characterized in that** the vibrator (15) comprises an electric motor (16), a cam (17) being fastened to the shaft of said electric motor.

5. The device according to one of the preceding claims, **characterized in that** a switching device, which can be adjusted by the user of the device, is provided in the housing (41) and produces the electrical connections between the energy source and the electrodes (7, 20), that the vibrator (15) is also connected to the electrical energy source by the switching device, that the switching device is provided with an electronic memory, in which the parameters of operating states to be selected are stored and can be selected by the user by means of a button (5), one of the parameters relating to the polarity of the electrodes, and **in that** the vibrator (15) is of the kind that operates independently of the polarity of the applied voltage.

6. The device according to one of the preceding claims, **characterized in that** a device for detecting a current flow through the second electrode (20), a switch in the connection of the drive of the vibrator to batteries, and a control device are provided, wherein the control device is configured in such a way that the switch is only closed when a current flows through the second electrode (20).

## Revendications

1. Appareil tenant dans une main, destiné au traitement à assistance électrique de la peau, comportant :
un boîtier (41),
une première électrode extérieure (7) sur le boîtier (41), qui est en contact avec la main lorsque l'utilisateur tient l'appareil dans la main pour en faire usage,
une deuxième électrode extérieure (20) sur le boîtier (41), que l'on peut placer sur la zone cutanée qui doit être traitée, et
une source de tension électrique sous la forme de batteries (30) à l'intérieur du boîtier (41), dont les pôles sont électriquement connectés aux électrodes (7, 20) lors du fonctionnement de l'appareil,
**caractérisé**
**en ce que** le boîtier est un boîtier (41) plat, doté d'une face inférieure et d'une face supérieure,
**en ce que** la deuxième électrode (20) se trouve sur la face inférieure du boîtier (41) en occupant quasiment l'ensemble de la face inférieure,
**en ce que** la première électrode (7) se situe en périphérie sur le bord extérieur de la face supérieure du boîtier (41),
**en ce qu'**à l'intérieur du boîtier se trouve un vibrateur (15) et
**en ce que** le vibrateur (15) est monté au centre au-dessus de la deuxième électrode (20) au moyen d'un socle sur la paroi inférieure du boîtier (41) et amène ainsi la paroi en vibration, conjointement avec la deuxième électrode (20) lorsqu'il est mis en service, de sorte que lors du fonctionnement de l'appareil, un effet de massage renforcé soit exercé sur la peau qui doit être traitée, ce qui a pour effet d'ouvrir les pores de la peau.

2. Appareil selon la revendication 1, **caractérisé en ce que** le vibrateur (15) est monté à proximité de la deuxième électrode extérieure (20).

3. Appareil selon la revendication 1 ou 2, **caractérisé en ce que** sur la face supérieure se trouvent un écran (6) et une touche (5), destinés à sélectionner un programme de traitement.

4. Appareil selon l'une quelconque des revendications précédentes, **caractérisé en ce que** le vibrateur (15) est constitué d'un moteur électrique (16) sur l'arbre duquel est fixé un excentrique (17).

5. Appareil selon l'une quelconque des revendications précédentes, **caractérisé en ce qu'**un dispositif de commutation réglable par l'utilisateur de l'appareil, qui établit les connexions électriques entre la source d'énergie et les électrodes (7, 20) est présent dans le boîtier (41), **en ce qu'**également le vibrateur (15) est connecté avec la source d'énergie électrique par l'intermédiaire du dispositif de commutation, **en ce que** le dispositif de commutation est muni d'une mémoire électronique dans laquelle les paramètres d'états de fonctionnement sélectionnables sont mémorisés qui peuvent être sélectionnés par l'utilisateur au moyen d'une touche (5), l'un des paramètres se rapportant à la polarité des électrodes, et **en ce que** le vibrateur (15) est du type à fonctionner indépendamment de la polarité de la tension appliquée.

6. Appareil selon l'une quelconque des revendications précédentes, caractérises-en ce que sont prévus un dispositif de détection d'une circulation de courant à travers la deuxième électrode (20), un interrupteur dans la connexion de l'entraînement du vibrateur avec les batteries et un système de commande, le système de commande étant aménagé de telle sorte que l'interrupteur ne soit fermé que lorsqu'un courant circule à travers la deuxième électrode (20).
